(19) Europäisches Patentamt
European Patent Office
Office européen des brevets

(11) Veröffentlichungsnummer: **0 112 479**
**B1**

(12) # EUROPÄISCHE PATENTSCHRIFT

(45) Veröffentlichungstag der Patentschrift: **10.08.88**

(21) Anmeldenummer: **83111345.1**

(22) Anmeldetag: **12.11.83**

(51) Int. Cl.⁴: **C 07 C 41/16,** C 07 C 85/00 // C07D213/74, C07D303/36, C07B39/00, C07B43/00, C07B45/00

(54) **Durchführung organischer Reaktionen in Siliconölen.**

(30) Priorität: **24.12.82 DE 3247994**
**08.03.83 DE 3308089**

(43) Veröffentlichungstag der Anmeldung:
**04.07.84 Patentblatt 84/27**

(45) Bekanntmachung des Hinweises auf die Patenterteilung:
**10.08.88 Patentblatt 88/32**

(84) Benannte Vertragsstaaten:
**DE FR GB IT NL**

(56) Entgegenhaltungen:
**DE-A-2 507 882**
**DE-A-2 642 833**
**FR-A-2 375 200**
**US-A-3 965 178**
**US-A-4 176 131**

**PATENTS ABSTRACTS OF JAPAN, Band 5, Nr. 124(C-66)(796), 11. August 1981**

(73) Patentinhaber: **Hüls Troisdorf Aktiengesellschaft, Postfach 11 65, D-5210 Troisdorf (DE)**

(72) Erfinder: **Suerken, Hans Peter, Kloosterwiel 9, NL-5301 WH Zaltbommel (NL)**
Erfinder: **Amort, Jürgen, Dr., Ubierstrasse 9, D-5210 Troisdorf- Sieglar (DE)**
Erfinder: **Hanisch, Horst, Dr., Fröbelweg 19, D-5202 Hennef/Sieg (DE)**
Erfinder: **van der Maas, Hendrikus, De Googerd 25, NL- 5305 CK Zuilichem (NL)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 112 479 B1

LIBER, STOCKHOLM 1988

**Beschreibung**

Die vorliegende Erfindung befaßt sich mit der Durchführung organischer Reaktionen in bestimmten Reaktionsmedien.

Es ist allgemein bekannt, organische Reaktionen in organischen Lösungsmitteln durchzuführen. Die wichtigsten Lösungsmittel sind aliphatische oder aromatische Kohlenwasserstoffe oder entsprechende Alkohole, einige Ketone, Ether, Ester und kurzkettige Chlorkohlenwasserstoffe. Ausschlaggebend für die Wahl eines Lösungsmittels als Reaktionsmedium ist die Löslichkeit der miteinander reagierenden Verbindungen in den Lösungsmitteln sowie die Löslichkeit des oder der Reaktionsprodukte in dem Lösungsmittel. Wichtig sind fernerhin die Flüchtigkeit, Brennbarkeit und die Verdunstungsgeschwindigkeit des Lösungsmittels für dessen Wahl als Reaktionsmedium. Weiterhin ist in nahezu allen Fällen die Wahl eines Lösungsmittels als Reaktionsmedium davon abhängig, inwieweit dieses mit den Reaktionspartnern oder den Reaktionsprodukten reagiert.

Es ist weiterhin bekannt, daß eine große Anzahl organischer Reaktionen in den oben genannten Lösungsmitteln nur mit unbefriedigenden Ausbeuten oder unter Bildung verunreinigter Endprodukte verläuft. Es bestand deshalb die Aufgabe, für die Durchführung organischer Reaktionen ein Reaktionsmedium zu finden, das ein gutes Lösungsvermögen für die Reaktionspartner oder für ein gewünschtes Endprodukt besitzt und in dem sich die gewünschten Umsetzungen so durchführen lassen, daß Nebenreaktionen kaum oder nur in untergeordnetem Maße auftreten und in dem möglichst reine Endprodukte erhalten werden. Dabei soll die durchzuführende Verfahrensweise in diesen gesuchten Lösungsmitteln möglichst einfach und ohne großen apparativen Aufwand durchführbar sein.

In Erfüllung dieser Aufgabe wurde nun ein Verfahren zur Durchführung von Reaktionen zwischen

a) Alkyhalogeniden und Aminen
oder
b) Alkyhalogeniden und Alkoholen, Alkoholaten oder Mercaptanen
oder
c) Aminen und aromatischen Sulfonsäuren oder Sulfonsäurechloriden unter Bildung von organischen Ammoniumsalzen
oder
d) Aminen und Verbindungen, die eine Meth- oder Ethoxyruppe in α-Stellung zu einer ungesättigten Gruppe besitzen
oder
e) Aminen und aromatischen Carbonsäureestern, deren Esterkomponente 1 bis 4 C-Atome besitzt, unter Bildung der entsprechenden Säureamide
oder
f) sekundären Aminen mit Chloracetalen

gefunden, das dadurch gekennzeichnet ist, daß die Umsetzung der Reaktionspartner in einem Siliconöl durchgeführt wird, dessen Viskosität zwischen 40 und 20 000 $mm^2/s$ (cSt) bei 25° C liegt, und das entweder ein oligomeres oder polymeres Siloxan, das die Strukturelemente

$$-\left[ \underset{X_2}{\underset{|}{Si}} - O \right]- ,$$

wobei X für den Methyl- oder Phenylrest steht, enthält, oder ein Trialkoxysilan, deren Estergruppen vollständig durch Trialkylsilylgruppen substituiert sind, wobei der Alkylrest 1 bis 4 C-Atome aufweisen kann, oder ein Tetrakis(trialkylsiloxy)silan ist.

Die Verwendung von Siliconölen als Lösungsmittel wird zwar auch schon in der JP-A-5 659 719 beschrieben. Dort handelt es sich aber um eine spezielle Reaktion, nämlich die thermische Zersetzung von Dicyclopentadien zu Cyclopentadien, für die Siliconöl als Lösungsmittel vorgeschlagen wird.

Auch Reaktionen von Organosiliciumverbindungen mit organischen Verbindungen sind bereits in Siloxan als Lösungsmittel vorgeschlagen worden (vgl. US-PS 4 176 131 und DE-OS 25 07 882). Bei diesen Verfahren sind jedoch nicht, wie bei dem beanspruchten Verfahren, beide Reaktionspartner organische Verbindungen.

Der Vorteil der erfindungsgemäßen Verfahrensweise gegenüber den Verfahren des Standes der Technik geht aus einem Vergleich mit dem Verfahren der US-PS 3 965 178 hervor. Dort wird auch die Umsetzung zwischen Aminen und Alkylhalogeniden beschrieben, wobei als Lösungsmittel jedoch Ethanol oder Ethylacetat genannt wird. Bei Verwendung dieser Lösungsmittel erhält man jedoch nur sehr geringe Ausbeuten.

Die erfindungsgemäße Verfahrensweise eignet sich besonders gut bei der Durchführung von Substitutionsreaktionen, bei denen Halogenwasserstoff, insbesondere Chlorwasserstoff, frei wird und bei der Umsetzung von Alkylhalogeniden mit Aminen, insbesondere tertiären Aminen, unter Bildung von quaternären Ammoniumsalzen. Die Reaktionspartner sind dabei einerseits Alkylchloride, bei denen die Alkylgruppen durch funktionelle Reste substituiert sein können, die mit den Siloxanen nicht reagieren und andererseits entweder Amine oder Alkohole bzw. Mercapatane. Allgemein laufen diese Substitutionsreaktionen nach den Gleichungen ab

$$R'Cl + R_2N{-}R'' \longrightarrow R'{-}\underset{R\ R}{\overset{+}{N}}{-}R''\quad Cl^- \qquad (I)$$

und

$$R'Cl + MZ{-}R'' \longrightarrow R'{-}Z{-}R'' + MCl \qquad (II)\ (M = Alkalimetall)$$

wobei R' für Alkyl mit vorzugsweise 1 bis 8 C-Atomen oder Phenyl, R für Wasserstoff oder $C_{1-4}$-Alkylreste und R" für H oder R' und Z für Sauerstoff oder Schwefel stehen kann. Anstelle von Cl kann auch Br oder J stehen.

Anstelle des Alkylhalogenids kann auch ein Chloracetal mit einem Amin, vorzugsweise einem sekundärem Amin, zur Reaktion gebracht werden. Das Amin wird dabei im Überschuß eingesetzt, um den freiwerdenden Chlorwasserstoff zu binden.

Bei der Herstellung von Verbindungen entsprechend diesen Gleichungen hat die Arbeitsweise in Siloxanen den Vorteil, daß man Produkte mit großer Reinheit und in großen Ausbeuten erhält. Bei der Verfahrensweise in den bisher eingesetzten Lösungsmitteln sind die Ausbeuten geringer und die erhaltenen Reaktionsprodukte fallen nicht in so reiner Form an. Beispiele für Verbindungen, die auf diese Weise hergestellt werden können, sind Oxiranmethanamin-N,N,N -trimethylchlorid (auch unter dem Namen Glycidyltrimethylammoniumchlorid bekannt), Tetrabutylammoniumbromid, Benzyltrimethylammoniumbromid.

Als Ausgangsverbindung für die Herstellung von organischen Ammoniumsalzen eignen sich aber auch aromatische Sulfonsäuren und Sulfonsäurechloride, die mit Aminen erfindungsgemäß umgesetzt werden können. Der aromatische Kern kann dabei durch Alkylgruppen oder durch Halogen substituiert sein. Dabei lassen sich sowohl die Sulfonamidsäuren als auch die tertiären Ammoniumsalze herstellen. Hier hat die erfindungsgemäße Verfahrensweise den Vorteil, daß das gewünschte Reaktionsprodukt in dem Siloxan als Feststoff ausfällt und somit leicht von den übrigen Reaktionspartner abgetrennt werden kann.

Die Umsetzung von Aminen mit Alkoxygruppen enthaltenden Verbindungen erfolgt vorzugsweise mit Verbindungen, die eine Meth- oder Ethoxygruppe in α-Stellung zu einer ungesättigten Gruppe enthalten. Beispiele für entsprechende Ausgangsverbindungen sind Methoximethylenmalonsäuredimethylester und Ethoximethylenmalonsäurediethylester.

Bei der Reaktion von Aminen mit Carbonsäureestern ist der Carbonsäureester vorzugsweise ein aromatischer Ester, dessen Esterkomponente 1 bis 4 C-Atome besitzt. Als Amin kann hier auch Ammoniak eingesetzt werden. Produkte, die auf diese Weise hergestellt werden können, sind z. B. Benzoesäuremethylamid (aus Benzoesäureestern und Monomethylamin) oder Toluylamid aus einem Toluylester und Ammoniak.

Die erfindungsgemäße Verfahrensweise in Siloxanen als Reaktionsmedium ermöglicht weiterhin die Durchführung von Phasenübergangsreaktionen, wobei als zweite Phase hauptsächlich die wässrige Phase dient. Als zweite Phase kann aber auch ein anderes, mit den Siloxanen nicht mischbares, organisches Lösungsmittel dienen.

Im Falle, daß einer der Reaktionspartner in dem Siliconöl nicht löslich ist, kann dieser entweder aufgeschmolzen werden und im geschmolzenen Zustand gelöst dem Siliconöl zugesetzt werden oder er wird in einem organischen Lösungsmittel gelöst dem Siliconöl zugesetzt. Anschließend kann das verwendete organische Lösungsmittel abdestilliert werden.

Bei den erfindungsgemäß einsetzbaren Siloxanen handelt es sich einerseits um Oligomere und Polymere auf Basis von Dimethyl, Methylphenyl- und Diphenylsiloxanen, die sowohl ringförmige als auch kettenförmige Struktur haben können, und andererseits um Tri- oder Tetraalkoxysilane, deren Estergruppierungen vollständig durch Trialkysilylgruppen substituiert sind. Auch Verzweigungen sind möglich. Sie dürfen mit Alkyl- oder Arylhalogeniden keine Reaktion eingehen und nach Möglichkeit keine funktionellen Gruppen besitzen. Weiterhin sollen sie sowohl bei Raumtemperatur als auch nach Möglichkeit bei Temperaturen bis zu etwa 200 °C, vorzugsweise bis 100°C, flüssig sein. Die Viskosität der einsetzbaren Siloxane, die auch als Siliconöle bezeichnet werden können, liegt bei 25°C zwischen 40 und etwa 20 000 $mm^2/s$, vorzugsweise zwischen 50 und 2 000 $mm^2/s$. Die überwiegende Zahl der im folgenden genannten Verbindungen erfüllt diese Bedingungen.

Zu den einsetzbaren Siliconölen zählen insbesondere solche Verbindungen, die als Strukturelemente die Gruppierung

$$\left[\underset{X_2}{Si} - O\right]$$

enthalten, wobei X für den Methyl- oder Phenylrest steht. Bevorzugt werden solche Verbindungen eingesetzt, bei denen mindestens der eine der beiden Reste X ein Phenylrest ist. Beispiele für Verbindungen, die unter diese Formel fallen, sind Hexamethyldisiloxan, 1,3-Dimethyl-1,3-diphenyldisiloxan, 1,1,3,3-Tetraphenyl-1,3-dimethyldisiloxan, 1,1,5,5-Tetraphenyl-1,3,3,5-tetramethyltrisiloxan, 1,1,3,5,5-Pentaphenyl-1,3,5-trimethyltrisiloxan, Polydimethylsiloxan, Polydimethyldiphenylsiloxan,

Polymethylphenyldiphenyldisiloxan, Polydiphenylsiloxan. Die Endgruppen der Polymeren sind vorzugsweise Trimethylsiloxygruppen.

Beispiele für Siliconöle mit Ringstruktur sind Hexamethyl-cyclotrisiloxan, Octamethylcyclotetrasiloxan, Decamethylcyclopentasiloxan oder Hexaphenylcyclotrisiloxan.

Auch Alkyltrialkoxysilane, deren Estergruppen vollständig durch Trialkylsilylgruppen (Alkyl mit 1 bis 4 C-Atomen) substituiert sind, können erfindungsgemäß als Reaktionsmedien eingesetzt werden. Solche Verbindungen und deren Herstellung sind beispielsweise in der DE-PS 26 42 833 beschrieben. Auch Tetrakis(trialkylsiloxy)silane, Verbindungen, die unter dem Namen "Schweizerkreuz" im Handel bekannt sind, können erfindungsgemäß eingesetzt werden. Diese Verbindungen können als Orthokieselsäureester aufgefaßt werden, bei denen die Alkylreste der Estergruppen durch Trialkylsilylgruppen ersetzt sind.

**Beispiel 1**

Es werden 94,9 g Epichlorhydrin (= 1,026 Mol) in 450 ml Hexamethyldisiloxan aufgelöst. In die Lösung werden mitten eines Gaseinleitungsrohres innerhalb 2,5 Stunden 5,9 g (= 1 Mol) Trimethylamin eingeleitet. Nach Ablauf einer Stunde fiel bereits das gewünschte Oxiranmetylamin-N,N,N -trimethylchlorid als Feststoff aus; eine Temperaturerhöhung trat nicht ein, die Reaktionstemperatur lag während der gesamten Umsetzung zwischen 20 und 25° C.

Nach Ablauf von 24 Stunden wurde der Feststoff über ein geschlossenes Filter abfiltriert, mit Hexamethyldisiloxan und daraufhin mit Methylethylketon ausgewaschen und anschließend im Vakuum getrocknet.

Der Epoxidgehalt des getrockneten Produktes lag bei 93 bis 94 %.

Zu der Mutterlauge wurde 1 Mol Epichlorhydrin hinzugefügt und in diese Lösung wiederum 59 g Trimethylamin eingeleitet. Nach Ablauf von 24 Stunden wurde das ausgefallene Endprodukt in der oben beschriebenen Weise abgetrennt und aufgearbeitet. Der erhaltene Feststoff hatte wiederum eine Reinheit von 93 bis 94 %.

**Beispiel 2**

in 378 g eines Polydimethylsiloxans mit einer Viskosität von 50 $mm^2/s$ bei 25° C wurden 138,7 g Epichlorhydrin gelöst bzw. suspendiert. Anschließend wurden 59 g Trimethylamin eingeleitet. Das Epichlorhydrin löste sich im Laufe des Einleitens vollständig in dem Polydimethylsiloxan auf und nach Ablauf von 20 Min. bildeten sich die ersten Kristalle von Oxiranmethylamin-N,N,N-trimethylchlorid. Nach Ablauf von 24 Stunden wurde der erhaltene Feststoff abfiltriert und wie im Beispiel 1 aufgearbeitet, wobei zuerst mit Polydimethylsiloxan ausgewaschen wurde.

Die Mutterlauge wurde in gleicher Weise behandelt. Auch hier bildeten sich die ersten Kristalle nach Ablauf von 20 Min. Nach Ablauf von 24 Stunden wurde wiederum der Niederschlag abfiltriert und wie oben beschrieben aufgearbeitet. Er hatte eine Reinheit von 92 bis 93 %. Die Ausbeute betrug 55 %, bezogen auf eingesetztes Trimethylamin.

Die verbleibende Mutterlauge wurde in gleicher Weise wie die Mutterlauge des ersten Ansatzes weiterverarbeitet. Sie führte zu einem Endprodukt mit der gleichen Reinheit; die Ausbeute, bezogen auf Trimethylamin, betrug jedoch 72 %.

**Beispiel 3**

Es wurde in gleicher Weise wie in den Beispielen 1 und 2 gearbeitet, jedoch wurde als Siliconöl ein Polymethylphenylsiloxan eingesetzt. Die Aufarbeitung des Niederschlags der ersten Umsetzung ergab eine Ausbeute von 37,4 %, bezogen auf Trimethylamin mit einer Reinheit von 93 bis 95 %. Die Aufarbeitung der Mutterlauge dieser ersten Umsetzung ergab eine Ausbeute von 92 %, bezogen auf eingesetztes Trimethylamin.

**Beispiel 4**

Herstellung von Tetrabutylammoniumbromid

Die Herstellung dieser Verbindung wird z. B, in der US-PS 39 65 178 beschrieben. Dabei wird in Acrylnitril als Lösungsmittel gearbeitet, wobei Ausbeuten von mehr als 50 % erhalten werden. Das Arbeiten mit Acrylnitril erfordert jedoch erhebliche Sicherheitsvorkehrungen, die den Prozeß unwirtschaftlich machen. Zur Umgehung dieser Nachteile wurde bereits vorgeschlagen, die Umsetzung in höheren Alkoholen, Estern oder Ketonen durchzuführen. Dabei erhielt man jedoch Ausbeuten, die nur um 50 % lagen, wobei teilweise Reaktionszeiten von etwa 48 Stunden notwendig waren.

Erfindungsgemäß wurden in 1000 g eines Polydimethylsiloxans mit einer Viskosität von 50 $mm^2/s$ 186 g (= 1 Mol) Tributylamin aufgelöst. Zu dieser Lösung wurde 1 Mol (= 137 g) Butylbromid, das leicht gefärbt war, hinzugefügt. Unter leichtem Rückfluß zu Beginn der Reaktion ließ man das Gemisch bei 150 bis 155° C während 20 Stunden reagieren. Daraufhin wurden die noch verbleibenden flüchtigen Bestandteile unter vermindertem Druck abgetrennt.

Das erhaltene Reaktionsgemisch wurde abgekühlt und der erhaltene Kristallbrei in

Wasser aufgenommen. Die untere Lösung von Tetrabutylammoniumbromid wurde abgetrennt und die vorhandene Farbe durch Auswaschen mit Toluol entfernt. Aus dem verbleibenden wäßrigen Lösung konnten durch Eindampfen 234,5 g eines weißen, reinen Endprodukts erhalten werden, das eine Reinheit von 99,5 bis 100 %, bezogen auf Br, besaß. Die Ausbeute betrug 72,6 %.

**Beispiel 5**

Herstellung von Benzyltriethylammoniumchlorid

In 200 g Polydimethylsiloxan entsprechend dem Beispiel 4 wurden 101 g Triethylamin (1 Mol) aufgelöst. In diese Lösung wurden 137 g (1 Mol) Benzylchlorid eingerührt. Nach einer Reaktionszeit von 5 Stunden bei 80° C hatte sich bereits eine große Menge Feststoff gebildet, die abfiltriert und mit Methylethylketon ausgewaschen wurde.

Nach dem Trocknen wurden 174 g des Benzyltriethylammoniumchlorids mit einer Reinheit von 99,4 bis 99,5 % erhalten. Die Ausbeute lag bei 76,3 %.

**Beispiel 6**

Unter Stickstoff wurden 600 g eines Methyl-Phenyl-Polysiloxans, das im Handel unter der Bezeichnung Siliconöl-PD-5 (Hersteller Bayer AG, Leverkusen) erhältlich ist, mit 216,4 g (= 1 Mol) Ethoxymethylenmalonsäurediethylester und 108 g (= 1 Mol) 6-Methyl-2-aminopyridin vermischt. Das Aminopyridin lag dabei im geschmolzenen Zustand vor, in dem es in dem Siliconöl löslich war. Das Gemisch wurde auf einer Reaktionstemperatur zwischen 90 und 100° C gehalten und der bei der dabei stattfindenden Reaktion gebildete Alkohol unter geringem Vakuum abdestilliert. Nachdem kein Alkohol mehr abdestillierte, wurde das Reaktionsmedium auf 60° abgekühlt.

In dem abgekühlten Siliconöl fiel der gewünschte Methylpyridylaminomethylenmalonsäurediethylester in Form von Kristallen aus, die abfiltiert, bei 20° gewaschen und getrocknet wurden. Die Ausbeute des so erhaltenen Produkts, das eine Reinheit von 99 % besaß, betrug 316 g = 96,9 %.

**Patentanspruch**

Verfahren zur Durchführung von Reaktionen zwischen

a) Alkylhalogeniden und Aminen
oder
b) Alkyhalogeniden und Alkoholen, Alkoholaten oder Mercaptanen
oder
c) Aminen und aromatischen Sulfonsäuren oder Sulfonsäurechloriden unter Bildung von organischen Ammoniumsalzen
oder
d) Aminen und Verbindungen, die eine Meth- oder Ethoxyruppe in α-Stellung zu einer ungesättigten Gruppe besitzen
oder
e) Aminen und aromatischen Carbonsäureestern, deren Esterkomponente 1 bis 4 C-Atome besitzt, unter Bildung der entsprechenden Säureamide
oder
f) sekundären Aminen mit Chloracetalen,

dadurch gekennzeichnet, daß die Umsetzung der Reaktionspartner in einem Siliconöl durchgeführt wird, dessen Viskosität zwischen 40 und 20 000 $mm^2/s$ (cSt) bei 25° C liegt, und das entweder ein oligomeres oder polymeres Siloxan, das die Strukturelemente

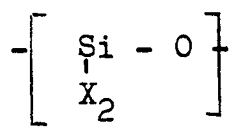

enthält, wobei X für den Methyl- oder Phenylrest steht, oder ein Trialkoxysilan, deren Estergruppen vollständig durch Trialkylsilylgruppen substituiert sind, wobei der Alkylrest 1 bis 4 C-Atome aufweisen kann, oder ein Tetrakis(trialkylsiloxy)silan ist.

**Claim**

Process for carrying out reactions between

a) alkyl halides and amines
or
b) alkyl halides and alcohols, alcoholates or mercaptans
or
c) amines and aromatic sulphonic acids or sulphonic acid chlorides with formation of organic ammonium salts
or
d) amines and compounds which possess a meth- or ethoxy group in the α-position to an unsaturated group
or
e) amines and aromatic carboxylic acid esters whose ester component possesses 1 to 4 C-atoms, with formation of the corresponding acid amides
or
f) secondary amines with chloracetals,

characterised in that the reaction of the reaction partners is carried out in a silicone oil whose viscosity lies between 40 and 20,000

$mm^2/s$ (cSt) at 25° C, and which is either an oligomeric or polymeric siloxane which contains the structural elements

$$-\left[\begin{array}{l} Si - O \\ \;| \\ X_2 \end{array}\right]-$$

wherein X stands for the methyl- or phenyl residue, or a trialkoxysilane, whose ester groups are substituted completely by trialkylsilyl groups, with the alkyl residue being able to possess 1 to 4 C-atoms, or a tetrakis(trialkylsiloxy)silane.

**Revendication**

Procédé pour la réalisation de réactions

a) entre des halogénures d'alkyle et des amines, ou

b) entre des halogénures d'alkyle et des alcools, des alcoolates ou des mercaptans, ou

c) entre des amines et des acides sulfoniques aromatiques ou des sulfochlorures avec formation de sels d'ammonium organiques, ou

d) entre des amines et des composés possédant un groupe méthoxy ou éthoxy en position-α par rapport à un groupe insaturé, ou

e) entre des amines et des esters carboxyliques aromatiques dont le composant ester possède 1 à 4 atomes de C, avec formation des amides correspondants ou

f) entre des amines secondaires et des chloracétals, caractérisé en ce que la réaction des partenaires réactionnels est conduite dans une huile de silicone dont la viscosité se situe entre 40 et 20 000 $mm^2/s$ (cSt) à 25° C et qui est un siloxane oligomère ou polymère renfermant les éléments structuraux

$$-\left[\begin{array}{l} Si - O \\ \;| \\ X_2 \end{array}\right]-$$

où X représente soit le reste méthyle ou phényle, un trialcoxysilane dont les groupes esters sont entièrement substitués par des groupes trialkylsilyles, le reste alkyle comportant 1 à 4 atomes de carbone, ou un tétrakis(trialkylsiloxy)silane.